# EUROPEAN PATENT APPLICATION

(11) **EP 4 516 256 A1**
(43) Date of publication of application: **05.03.2025**
(21) Application number: 23795970.5
(22) Date of filing: 24.03.2023
(51) Int. Cl.: A61B 50/20

(54) **HOOK FOR MEDICAL DEVICES, AND MEDICAL DEVICE**

(30) Priority: 26.04.2022 JP 2022072352
(71) Applicant: HOYA CORPORATION, Shinjuku-ku Tokyo 160-8347 (JP)
(72) Inventor: SUGITA, Noriyuki, Tokyo 160-8347 (JP)
(74) Representative: Schaumburg und Partner Patentanwälte mbB
(86) International application number: PCT/JP2023/011770
(87) International publication number: WO 2023/210223

(57) **Abstract**

This hook for medical devices is intended to be attached to a medical device having a tubular part that is inserted in a body cavity, and the hook is configured such that an insertion opening to which the tubular part can be inserted is formed, a peripheral part of the insertion opening is elastically deformed so as to allow the insertion opening to tightly adhere to the tubular part inserted thereinto, and the tubular part is held by frictional resistance generated at a tightly adhered part between the peripheral part of the insertion opening and the tubular part.

## Description

### Technical Field

The present invention relates to a medical-device hook and a medical device.

### Background Art

Known has been a medical device including a tubular portion to be inserted into a body lumen. For example, Patent Literature 1 describes a specific configuration of such a type of medical device.

The medical device described in Patent Literature 1 includes an operation body that a surgical operator (medical doctor) operates, in which the operation body has a base end portion to which a hook is attached. A case where the tubular portion is laid on the surface of a floor with the medical device suspended by hanging the hook on a hanger is unfavorable in terms of hygiene. In addition, for example, the surgical operator is likely to step on the tubular portion, leading to damage of the tubular portion.

Thus, the medical device described in Patent Literature 1 has, on its operation body, a locking groove for locking the tubular portion. The tubular portion is hung on the locking groove such that the tubular portion is suspended. Thus, the tubular portion requires no laying on the surface of a floor.

### Citation List

### Patent Literature

Patent Literature 1: JP S63-166209 U1

### Summary of Invention

### Technical Problem

In Patent Literature 1, in a case where the tubular portion has a large self-weight, due to a large load to the tubular portion through a portion in contact with the locking groove, the tubular portion is likely to buckle. In Patent Literature 1, in a case where the tubular portion has a small self-weight, due to a small degree of hanging on the locking groove, the tubular portion is likely to drop from the locking groove.

The present invention has been made in consideration of the above situation, and an object of the present invention is to provide a medical-device hook favorable for holding a tubular portion of a medical device having been suspended and a medical device including such a medical-device hook.

### Solution to Problem

According to an embodiment of the present invention, provided is a medical-device hook to be attached to a medical device including a tubular portion to be inserted into a body lumen, the medical-device hook including: an inset opening allowing the tubular portion to be inset into the inset opening; and a surrounding portion around the inset opening, the surrounding portion being configured to deform elastically to come in close contact with the tubular portion inset in the inset opening such that the tubular portion is held due to frictional resistance on a closely contacted portion between the surrounding portion around the inset opening and the tubular portion.

In the medical-device hook according to the embodiment of the present invention, when the medical-device hook is hung on a hanger, the surrounding portion around the inset opening may deform elastically, due to a weight of the medical device, to come in close contact with the tubular portion inset in the inset opening such that the tubular portion is held due to the frictional resistance on the closely contacted portion.

In the medical-device hook according to the embodiment of the present invention, when the tubular portion is inset into the inset opening, the surrounding portion around the inset opening may deform elastically to come in close contact with the tubular portion such that the tubular portion is held due to the frictional resistance on the closely contacted portion.

In the medical-device hook according to the embodiment of the present invention, the inset opening may include a hole or a slit.

In the medical-device hook according to the embodiment of the present invention, the inset opening includes, for example, a hole having a diameter larger than an outer diameter of the tubular portion.

In the medical-device hook according to the embodiment of the present invention, the inset opening includes, for example, a slit having a width narrower than an outer diameter of the tubular portion.

In the medical-device hook according to the embodiment of the present invention, for example, the medical-device hook is formed of elastomer resin.

According to an embodiment of the present invention, provided is a medical device including: a tubular portion to be inserted into a body lumen; a grip portion connected to a base end portion of the tubular portion; and the medical-device hook described above, the medical-device hook being attached to a base end portion of the grip portion.

In the medical device according to the embodiment of the present invention, for example, the medical-device hook is attached to the grip portion so as to pose at a slant with respect to a direction of gravity when the medical-device hook is hung on a hanger.

### Advantageous Effects of Invention

According to an embodiment of the present invention, provided are a medical-device hook favorable for holding a tubular portion of a medical device having been suspended and a medical device including such a medical-device hook.

### Brief Description of Drawings

Fig. 1 illustrates the entirety of an endoscope treatment tool to which a hook according to a first embodiment of the present invention is attached.
Fig. 2 illustrates a state where the endoscope treatment tool is suspended with the hook hung on a hanger in the first embodiment of the present invention.
Fig. 3 is an arrow view of the hook according to the first embodiment of the present invention.
Fig. 4A illustrates a state where a tubular distal tip of the endoscope treatment tool is inset in an inset hole of the hook in the first embodiment of the present invention.
Fig. 4B illustrates the state of the inset hole with the hook hung on the hanger in the first embodiment of the present invention.
Fig. 5 is an arrow view of a hook according to a second embodiment of the present invention.
Fig. 6A illustrates how a tubular distal tip of an endoscope treatment tool is inset into a slit of the hook in the second embodiment of the present invention.
Fig. 6B illustrates how the tubular distal tip of the endoscope treatment tool is inset into the slit of the hook in the second embodiment of the present invention.
Fig. 7 is an arrow view of a hook according to a third embodiment of the present invention.
Fig. 8A illustrates how a tubular distal tip of an endoscope treatment tool is inset into a slit of the hook in the third embodiment of the present invention.
Fig. 8B illustrates how the tubular distal tip of the endoscope treatment tool is inset into the slit of the hook in the third embodiment of the present invention.

### Description of Embodiments

Medical-device hooks and medical devices according to embodiments of the present invention will be described below with reference to the drawings.

A medical-device hook according to the present embodiment is attached to a medical device including a tubular portion to be inserted into a body lumen. As an example, the medical-device hook is attached to an endoscope treatment tool including a high-frequency knife that excises, for example, a mucous membrane due to passage of a high-frequency current in endoscopic submucosal dissection (ESD).

A medical device to which the medical-device hook according to the present embodiment is to be attached is not limited to such an endoscope treatment tool. The medical-device hook may be attached to a different type of medical device including a tubular portion to be inserted into a body lumen (e.g., an endoscope).

### [First Embodiment]

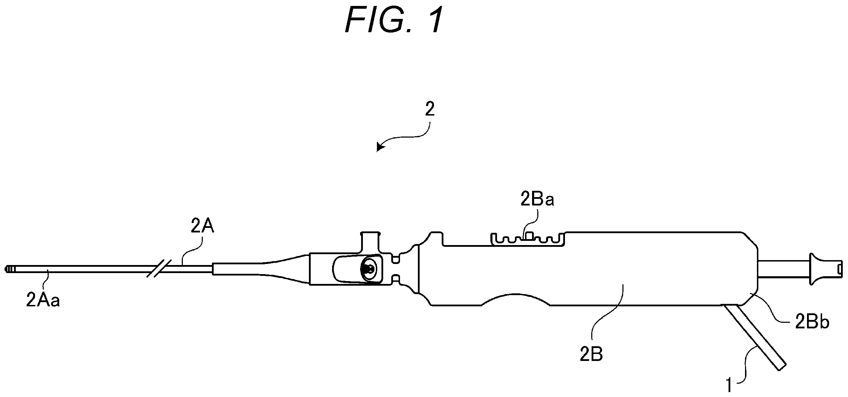

Fig. 1 illustrates the entirety of an endoscope treatment tool 2 to which a hook 1 according to a first embodiment of the present invention is attached. The hook 1 is an exemplary medical-device hook.

The endoscope treatment tool 2 includes a tubular portion 2A and a grip portion 2B, in addition to the hook 1.

The tubular portion 2A serves as a portion to be inserted into a body lumen (more particularly, a portion that has passed through a channel of an endoscope and is to be inserted into a body lumen) and has flexibility to deform following organs in a body lumen. The tubular portion 2A has a distal tip 2Aa (hereinafter, referred to as a "tubular distal tip 2Aa") in which a high-frequency knife is provided.

The grip portion 2B serves as a portion to be gripped by a surgical operator and is connected to the base end portion of the tubular portion 2A. The grip portion 2B is provided with an operation unit 2Ba to be operated by a surgical operator.

In response to an operation to the operation unit 2Ba by a surgical operator, a change is made in the amount of protrusion of the high-frequency knife to the tip face of the tubular distal tip 2Aa. The surgical operator operates the operation unit 2Ba, in accordance with procedure details (e.g., marking, local injection, incision, exfoliation, or hemostasis), to cause the high-frequency knife to protrude from the tip face of the tubular distal tip 2Aa by an appropriate amount.

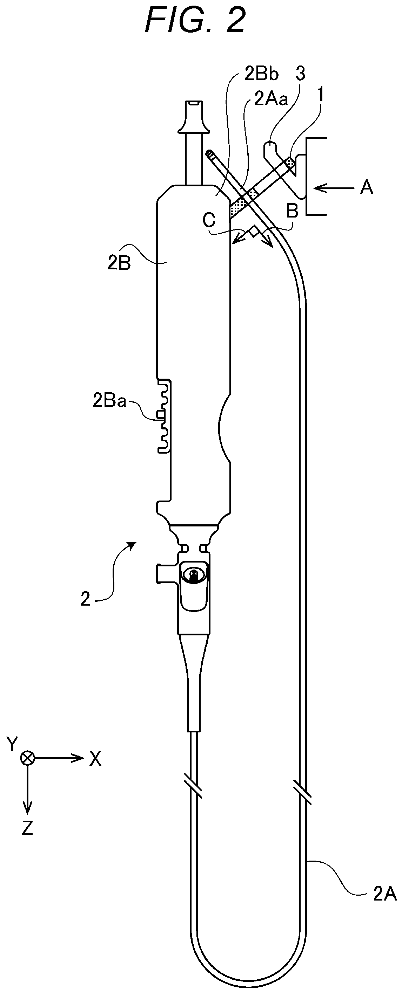

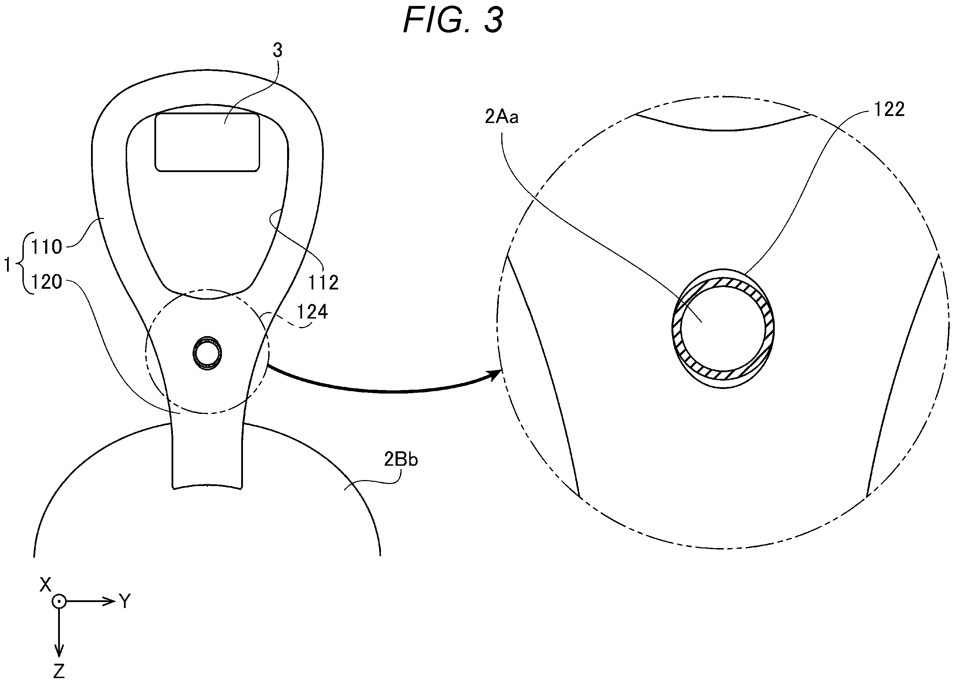

Fig. 2 illustrates a state where the endoscope treatment tool 2 is suspended with the hook 1 hung on a hanger 3. Fig. 3 is an arrow view of the hook 1 hung on the hanger 3 viewed in the direction of an arrow A in Fig. 2.

In the following description, the up-and-down direction is defined as a Z direction and two directions orthogonal to the Z direction and orthogonal to each other are defined as an X direction and a Y direction. The X direction, the Y direction, and the Z direction orthogonal to each other form a left-handed system. The positive Z direction corresponds to the direction of gravity.

The hook 1 is a resin molded product including a body portion 110 and a connection portion 120 integrally formed together. The hook 1 is formed of elastomer resin, such as silicone rubber or fluororubber. Thus, the hook 1 deforms elastically depending on an applied load.

The body portion 110 is annular in shape and has a hole 112. Hanging is performed on the hanger 3 through the hole 112.

The connection portion 120 connects the body portion 110 and the grip portion 2B. Specifically, the connection portion 120 is shaped like a flat plate that is wide laterally (in the Y direction, referring to Fig. 3). The connection portion 120 has a front end (on the negative side in the Z direction, referring to Fig. 3) integrated with the body portion 110 and a base end portion (on the positive side in the Z direction, referring to Fig. 3) embedded in a base end portion 2Bb of the grip portion 2B.

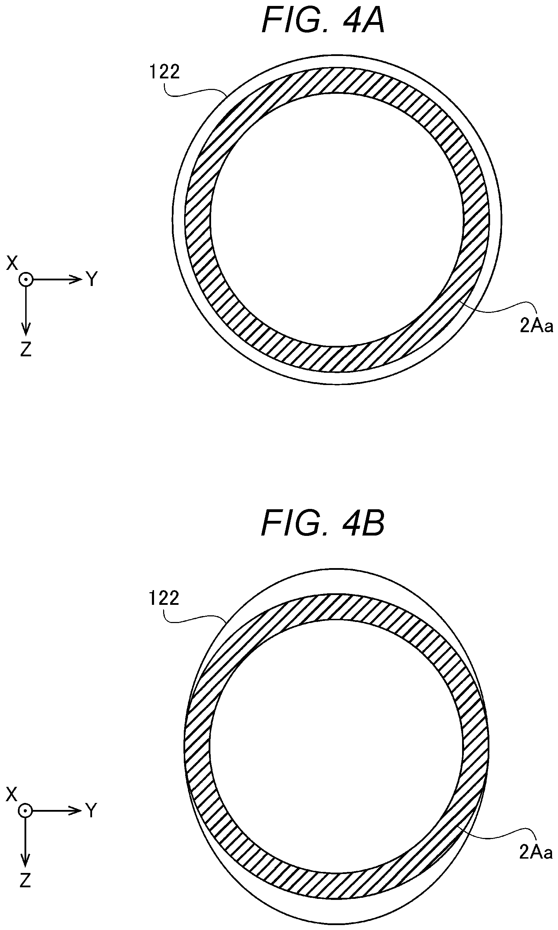

An inset hole 122 as an exemplary inset opening is located at the center in the width direction of the connection portion 120 (at the center in the Y direction, referring to Fig. 3) and below the hole 112 in a case where the hook 1 is hung on the hanger 3. The inset hole 122 is a circular hole having a diameter larger than the outer diameter of the tubular distal tip 2Aa of the endoscope treatment tool 2 (referring to Fig. 4A described later). That is, the tubular distal tip 2Aa can be inset into the inset hole 122.

Note that the inset hole 122 is not limited to such a circular hole and thus may be a hole in any shape (e.g., an elliptical hole or a polygonal hole).

For convenience, a surrounding portion around the inset hole 122 is referred to as a "surrounding portion 124".

The surgical operator or any other health professional (e.g., a physician assistant or a nurse) hangs the hook 1 on the hanger 3 provided on an indoor wall face to suspend the endoscope treatment tool 2. Hereinafter, anyone who suspends the endoscope treatment tool 2 is generically referred to as a worker.

The worker insets the tubular distal tip 2Aa into the inset hole 122 and then hangs the hook 1 on the hanger 3.

Fig. 4A illustrates a state where the tubular distal tip 2Aa is inset in the inset hole 122. As illustrated in Fig. 4A, since the diameter of the inset hole 122 is larger than the outer diameter of the tubular distal tip 2Aa, the worker can easily inset the tubular distal tip 2Aa into the inset hole 122.

Fig. 4B illustrates the state of the inset hole 122 with the hook 1 hung on the hanger 3. When the hook 1 is hung on the hanger 3, due to the weight of the endoscope treatment tool 2, almost the entirety of the hook 1 including the surrounding portion 124 around the inset hole 122 deforms elastically to extend slightly in the Z direction.

Due to elastic deformation of the surrounding portion 124, the inset hole 122 deforms from its circular shape to an elliptical shape having its major axis and minor axis, respectively, in the Z direction and the Y direction, as illustrated in Fig. 4B. Part of the surrounding portion 124 elastically deformed such that the inset hole 122 narrows in the Y direction is in close contact with the tubular distal tip 2Aa inset in the inset hole 122. Such close contact between the surrounding portion 124 and the tubular distal tip 2Aa causes adequate frictional resistance therebetween. Thus, the tubular distal tip 2Aa is firmly held by the surrounding portion 124 without dropping from the inset hole 122.

As above, the hook 1 allows the surrounding portion 124 around the inset hole 122 to deform elastically due to the self-weight of the endoscope treatment tool 2 having been suspended. Such elastic deformation of the surrounding portion 124 causes the surrounding portion 124 to come in close contact with the tubular distal tip 2Aa inset in the inset hole 122, so that the tubular distal tip 2Aa is held due to frictional resistance on the closely contacted portion between the surrounding portion 124 and the tubular distal tip 2Aa.

Even in a case where the endoscope treatment tool 2 has a large self-weight, the surrounding portion 124 and the tubular distal tip 2Aa come in close surface contact. Thus, the load on the tubular distal tip 2Aa is more dispersed, in comparison to the configuration described in Patent Literature 1. Thus, the tubular distal tip 2Aa is less likely to buckle. Even in a case where the endoscope treatment tool 2 has a small self-weight, the surrounding portion 124 and the tubular distal tip 2Aa come in close surface contact. In addition, the hook 1 is made of elastomer resin large in static friction coefficient. Thus, adequate frictional resistance occurs between the surrounding portion 124 and the tubular distal tip 2Aa. Thus, the tubular distal tip 2Aa is less likely to drop from the inset hole 122.

As illustrated in Fig. 2, the hook 1 is attached to the base end portion 2Bb of the grip portion 2B so as to pose at a slant with respect to the direction of gravity (with respect to the positive Z direction) when the hook 1 is hung on the hanger 3. Thus, in practice, the hook 1 does not deform elastically to extend only in the Z direction but deforms elastically, due to the self-weight of the endoscope treatment tool 2, not only to bend in the direction of an arrow B resulting from composition of the positive Z direction and the positive X direction but also to extend in the direction of an arrow C resulting from composition of the positive Z direction and the negative X direction.

The surrounding portion 124 deforms elastically to bend, and thus the load from the surrounding portion 124 to the tubular distal tip 2Aa is dispersed in various directions through the closely contacted portion between the surrounding portion 124 and the tubular distal tip 2Aa. From this regard, the tubular distal tip 2Aa is less likely to drop from the inset hole 122.

### [Second Embodiment]

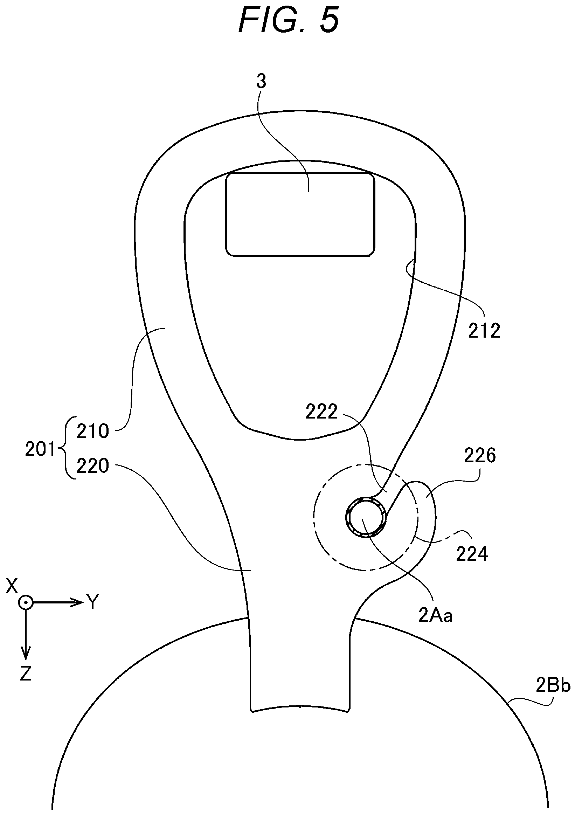

Fig. 5 illustrates a hook 201 according to a second embodiment of the present invention. Note that hooks according to the following embodiments including the second embodiment are each attached to an endoscope treatment tool 2, similarly to the first embodiment. Fig. 5 is an arrow view of the hook 201 hung on a hanger 3, similarly to Fig. 3.

Similarly to the hook 1 according to the first embodiment, the hook 201 is a resin molded product including a body portion 210 and a connection portion 220 integrally formed together, and deforms elastically depending on an added load.

Similarly to the body portion 110 according to the first embodiment, the body portion 210 is annular in shape and has a hole 212.

Similarly to the connection portion 120 according to the first embodiment, the connection portion 220 is shaped like a flat plate that is wide laterally. The connection portion 220 has a front end integrated with the body portion 210 and a base end portion embedded in a base end portion 2Bb of a grip portion 2B.

The hook 201 includes a protrusion 226 protruding laterally from the connection portion 220 near the boundary between the body portion 210 and the connection portion 220. The protrusion 226 extends along an outer circumferential face 210A of the body portion 210. In the second embodiment, a slit 222 is provided as an exemplary inset opening between the outer circumferential face 210A of the body portion 210 and the protrusion 226.

The slit 222 includes a linear slit portion (linear portion 222a) and a circular slit portion (circular portion 222b). The width of the linear portion 222a is narrower than the outer diameter of a tubular distal tip 2Aa, and the diameter of the circular portion 222b is smaller than the outer diameter of the tubular distal tip 2Aa. For convenience, a surrounding portion around the circular portion 222b is referred to as a "surrounding portion 224".

The hook 201 deforms elastically depending on an added load. Thus, a worker can easily inset the tubular distal tip 2Aa into the slit 222.

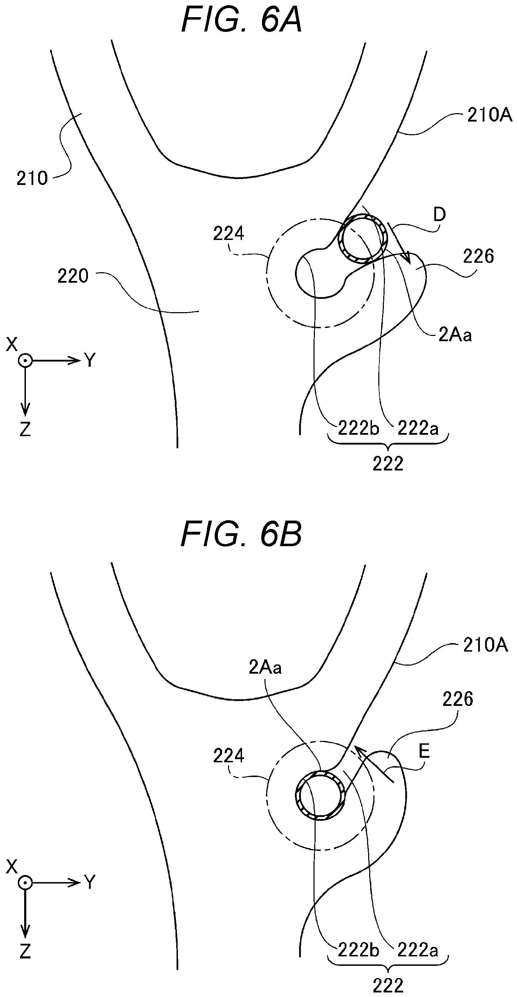

Figs. 6A and 6B illustrate how the tubular distal tip 2Aa is inset into the slit 222. As illustrated in Fig. 6A, when the tubular distal tip 2Aa is inset in the linear portion 222a of the slit 222, the protrusion 226 is pushed and moved in the direction of an arrow D, so that the linear portion 222a widens. As illustrated in Fig. 6B, when the tubular distal tip 2Aa is inset to the circular portion 222b of the slit 222, the load to the protrusion 226 due to the tubular distal tip 2Aa drops to almost zero. Thus, the protrusion 226 moves in the direction of an arrow E, so that the linear portion 222a gets back an almost original width.

The diameter of the circular portion 222b is smaller than the outer diameter of the tubular distal tip 2Aa. Thus, the surrounding portion 224 around the circular portion 222b deforms elastically such that the circular portion 222b is pushed and widened along the outer diameter of the tubular distal tip 2Aa.

Due to the above elastic deformation, the surrounding portion 224 comes in close contact with the tubular distal tip 2Aa inset in the circular portion 222b. Such close contact between the surrounding portion 224 and the tubular distal tip 2Aa causes adequate frictional resistance therebetween. Thus, the tubular distal tip 2Aa is firmly held by the surrounding portion 224 without dropping from the circular portion 222b.

The width of the linear portion 222a is narrower than the diameter of the circular portion 222b. Thus, even in a case where the tubular distal tip 2Aa comes off the circular portion 222b, the tubular distal tip 2Aa is less likely to drop through the linear portion 222a.

As above, the hook 201 allows the surrounding portion 224 to deform elastically following the tubular distal tip 2Aa inset in the slit 222. Due to such elastic deformation of the surrounding portion 224, the surrounding portion 224 and the tubular distal tip 2Aa come in close contact, so that the tubular distal tip 2Aa is held due to frictional resistance on the closely contacted portion between the surrounding portion 224 and the tubular distal tip 2Aa.

In the second embodiment, even in a case where the endoscope treatment tool 2 has a large self-weight, the surrounding portion 224 and the tubular distal tip 2Aa come in close surface contact. Thus, the load on the tubular distal tip 2Aa is more dispersed, in comparison to the configuration described in Patent Literature 1. Thus, the tubular distal tip 2Aa is less likely to buckle. Even in a case where the endoscope treatment tool 2 has a small self-weight, the surrounding portion 224 and the tubular distal tip 2Aa come in close surface contact. In addition, the hook 201 is made of elastomer resin large in static friction coefficient. Thus, adequate frictional resistance occurs between the surrounding portion 224 and the tubular distal tip 2Aa. Thus, the tubular distal tip 2Aa is less likely to drop from the slit 222.

### [Third Embodiment]

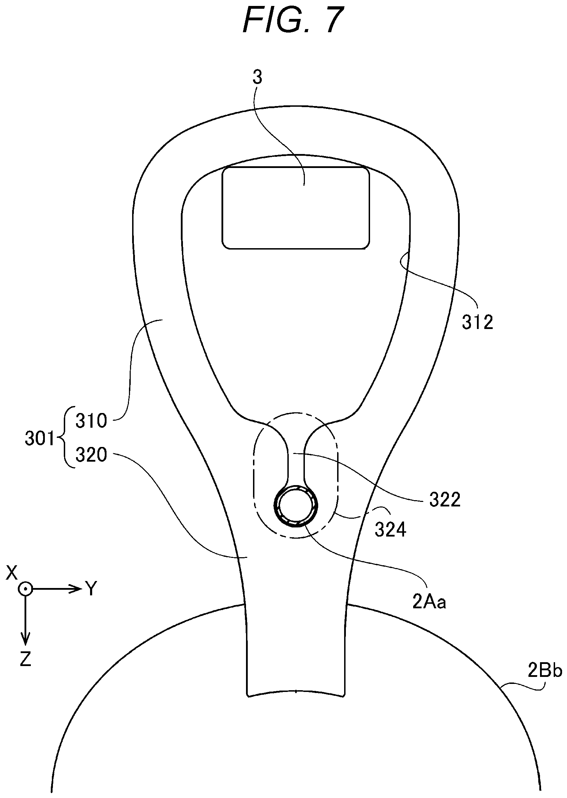

Fig. 7 illustrates a hook 301 according to a third embodiment of the present invention. Fig. 7 is an arrow view of the hook 301 hung on a hanger 3, similarly to Fig. 3.

Similarly to the hook 1 according to the first embodiment, the hook 301 is a resin molded product including a body portion 310 and a connection portion 320 integrally formed together, and deforms elastically depending on an added load.

Similarly to the body portion 110 according to the first embodiment, the body portion 310 is annular in shape and has a hole 312.

Similarly to the connection portion 120 according to the first embodiment, the connection portion 320 is shaped like a flat plate that is wide laterally. The connection portion 320 has a front end integrated with the body portion 310 and a base end portion embedded in a base end portion 2Bb of a grip portion 2B.

The body portion 310 has a slit 322 below the hole 312 (more particularly, located below the hole 312 when the hook 301 is hung on the hanger 3).

The slit 322 includes a linear slit portion (linear portion 322a) and a circular slit portion (circular portion 322b). The linear portion 322a extends longitudinally (in the Z direction, referring to Fig. 7). The linear portion 322a has a front end (on the negative side in the Z direction, referring to Fig. 7) in communication with the hole 312 and a base end (on the positive side in the Z direction, referring to Fig. 7) in communication with the circular portion 322b.

The width of the linear portion 322a is narrower than the outer diameter of a tubular distal tip 2Aa, and the diameter of the circular portion 322b is larger than the outer diameter of the tubular distal tip 2Aa. For convenience, a surrounding portion around the slit 322 is referred to as a "surrounding portion 324".

The hook 301 deforms elastically depending on an added load. Thus, a worker can easily inset the tubular distal tip 2Aa into the slit 322.

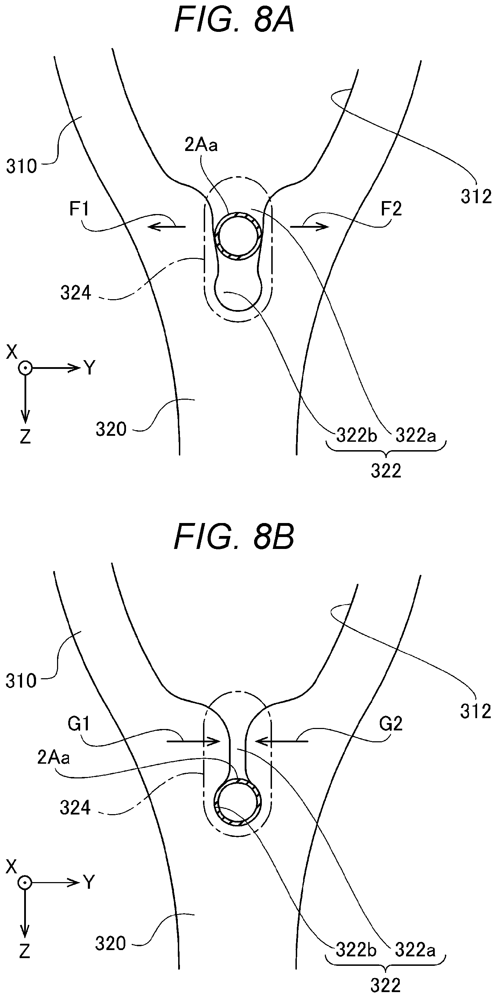

Figs. 8A and 8B illustrate how the tubular distal tip 2Aa is inset into the slit 322. As illustrated in Fig. 8A, when the tubular distal tip 2Aa is inset in the linear portion 322a of the slit 322, the surrounding portion 324 determining the linear portion 322a is pushed and widened in the Y direction, as indicated with arrows F1 and F2. As illustrated in Fig. 8B, when the tubular distal tip 2Aa is inset to the circular portion 322b of the slit 322, the surrounding portion 324 determining the linear portion 322a gets back the original shape, as indicated with arrows G1 and G2.

In the third embodiment, due to the self-weight of the endoscope treatment tool 2, the hook 301 deforms elastically such that its entirety extends in the Z direction, so that the linear portion 322a and the circular portion 322b deform. Specifically, due to elastic deformation of the surrounding portion 324, the linear portion 322a deforms not only to extend in the Z direction but also to narrow in the Y direction and additionally the circular portion 322b deforms elliptically, similarly to the inset hole 122 in the first embodiment.

Due to a decrease in the width of the linear portion 322a, even in a case where the tubular distal tip 2Aa comes off the circular portion 322b, the tubular distal tip 2Aa is less likely to drop through the linear portion 322a. Due to elliptical deformation of the circular portion 322b, the tubular distal tip 2Aa and the surrounding portion 324 comes in close contact. Thus, the tubular distal tip 2Aa is less likely to drop from the slit 322.

Note that the diameter of the circular portion 322b may be smaller than the outer diameter of the tubular distal tip 2Aa. In this case, when the tubular distal tip 2Aa is inset into the circular portion 322b, the surrounding portion 324 deforms elastically such that the circular portion 322b is pushed and widened along the outer diameter of the tubular distal tip 2Aa.

Due to the above elastic deformation, the surrounding portion 324 comes in close contact with the tubular distal tip 2Aa inset in the circular portion 322b. As described above, a load that causes the circular portion 322b to deform elliptically is further applied from the surrounding portion 324 to the tubular distal tip 2Aa. Thus, in the third embodiment, the tubular distal tip 2Aa is further firmly held.

Exemplary embodiments of the present invention have been described above. Embodiments of the present invention are not limited to the embodiments described above, and thus various modifications can be made without departing from the scope of the technical idea of the present invention. For example, any appropriate combination of the exemplary embodiments herein and conceivable embodiments is to be included in embodiments of the present invention.

## Claims

1. A medical-device hook to be attached to a medical device including a tubular portion to be inserted into a body lumen, the medical-device hook comprising:
an inset opening allowing the tubular portion to be inset into the inset opening; and
a surrounding portion around the inset opening, the surrounding portion being configured to deform elastically to come in close contact with the tubular portion inset in the inset opening such that the tubular portion is held due to frictional resistance on a closely contacted portion between the surrounding portion around the inset opening and the tubular portion.

2. The medical-device hook according to claim 1, wherein
when the medical-device hook is hung on a hanger, the surrounding portion around the inset opening deforms elastically, due to a weight of the medical device, to come in close contact with the tubular portion inset in the inset opening such that the tubular portion is held due to the frictional resistance on the closely contacted portion.

3. The medical-device hook according to claim 1, wherein
when the tubular portion is inset into the inset opening, the surrounding portion around the inset opening deforms elastically to come in close contact with the tubular portion such that the tubular portion is held due to the frictional resistance on the closely contacted portion.

4. The medical-device hook according to claim 2 or 3, wherein
the inset opening includes a hole or a slit.

5. The medical-device hook according to claim 2 or 3, wherein
the inset opening includes a hole having a diameter larger than an outer diameter of the tubular portion.

6. The medical-device hook according to claim 2 or 3, wherein
the inset opening includes a slit having a width narrower than an outer diameter of the tubular portion.

7. The medical-device hook according to any one of claims 1 to 3, wherein
the medical-device hook is formed of elastomer resin.

8. A medical device comprising:
a tubular portion to be inserted into a body lumen;
a grip portion connected to a base end portion of the tubular portion; and
the medical-device hook according to any one of claims 1 to 3, the medical-device hook being attached to a base end portion of the grip portion.

9. The medical device according to claim 8, wherein
the medical-device hook is attached to the grip portion so as to pose at a slant with respect to a direction of gravity when the medical-device hook is hung on a hanger.
